# EUROPEAN PATENT APPLICATION

(11) **EP 3 799 056 A1**
(43) Date of publication of application: **31.03.2021**
(21) Application number: 19199116.5
(22) Date of filing: 24.09.2019
(51) Int. Cl.: G16H 10/60

(54) **CLOUD-BASED PATIENT DATA EXCHANGE**

(71) Applicant: Siemens Healthcare GmbH, 91052 Erlangen (DE)
(72) Inventor: MANUEL, Sujith, 91052 Erlangen (DE); ROSENBAUM, Ute, 87437 Kempten (DE); SPIES, Carsten, 91088 Bubenreuth (DE)

(57) **Abstract**

The present invention is directed to a method for sharing medical data sets, with the steps of: receiving medical data sets from a medical system (10); receiving associated information from a database (20) separated from the medical system (10), wherein the associated information provides information associated to the medical data sets; uploading the medical data sets to a cloud platform (40); processing the associated information and the medical data sets to identify one or more entitled users (50), the entitled users being entitled to access a respective medical data set; retrieving additional user information of the entitled users (50); and granting the entitled users (50) access to the respective medical data set in the cloud platform (40) on the basis of the additional user information.

## Description

The amount of digital data collected in healthcare experienced an ever-increasing rise in the recent decades. This pertains to administrative data on the one hand as well as to medical data such as patients' test results or radiology images on the other hand.

To handle these data, healthcare informatics relies on a plurality of information and storage systems which help and support clinicians and clinical staff members. While databases such as hospital information systems (HIS), radiology information systems (RIS), clinical information systems (CIS), laboratory information systems (LIS) or cardiovascular information systems (CVIS) focus on the administration needs of the hospitals, storage and archiving medical systems such as the picture archiving and communicating system (PACS) enable an economical storage and convenient access to medical data.

Of note, the different systems and databases used are typically separated from one another and often rely on different standards and formats for storing and forwarding data. For instance, the established standard for transmitting data pertaining to hospital information systems is HL7, while the universal format for PACS image storage and transfer is DICOM (Digital Imaging and Communications in Medicine). By consequence, data streams from the different systems and databases are not readily compatible even though they usually relate to mutually complementary information which has to be combined in clinical workflows. This mutually complementary information may, for instance, relate to the patients' test results on the one hand and to information associated thereto, such as billing information, patients' health histories, or information about referring physicians, on the other hand. While the former information is typically stored and transmitted in the form of dedicated medical data sets, the latter information is archived in the form of associated information in separate databases.

The architecture used at present is sufficient as long as each administrative unit (such as a hospital, an imaging center or a radiology practice) works with its own data. However, the current systems often reach their limits when information needs to be handed over to the outside of these organizations - a scenario which arises whenever a patient is referred to another physician or another hospital, for instance.

One issue in this regard is that medical files are often subject to proprietary formatting applied by a combination of equipment manufacturers as well as individual computer networks within the organizations. Another issue relates to the inherent sensitivity of medical data which makes medical entities generally very reluctant to grant direct access to databases within their own control as this would raise important patient privacy concerns.

Moreover, problems may arise from the inherent incompatibility of the data sources needed for handling an efficient data transfer. As mentioned, databases for storing personal information, such as address details or contact information (required for forwarding medical data to a patient), typically rely on different data standards than the systems storing the medical data sets to be forwarded. In other words, various different databases have to be accessed manually to retrieve the required information for sharing medical data sets.

What is more, already the information who is actually entitled to use a given medical data set is usually spread over different data sources. This may depend on the practices of the individual organization, the case under consideration or even on the entitled persons as such. For example, the patient, as entitled user, is typically (but not necessarily) derivable from medical data set as such. By contrast, detailed information pertaining to a referring physician, for instance, is usually not contained in the medical data set. This makes any straightforward automatic queries elusive and hampers the integration into existing clinical workflows.

All this has the striking consequence that even nowadays medical data - and, in particular, medical imaging data - is still exchanged physically in the form of digital memory storage media, such as CDs, DVDs or memory sticks.

It is thus an object of the present invention to provide means and/or methods which allow for an improved way of sharing medical data with entitled users outside of the clinical organizations such as referring physicians or the patients themselves. Particularly, it is an object of the present invention to provide means and/or methods that allow for a seamless integration of this process into existing clinical workflows which are generally compatible to existing clinical hard- and software equipment.

This object is solved by a method for sharing medical data sets, corresponding system, corresponding computer-program product and computer-readable storage medium according to the independent claims. Alternative embodiments are object of the dependent claims and are set out below.

In the following, the technical solution according to the present invention is described with respect to the claimed apparatuses as well as with respect to the claimed methods. Features, advantages or alternative embodiments described herein can likewise be assigned to other claimed objects and vice versa. In other words, claims addressing the inventive method can be improved by features described or claimed with respect to the apparatuses. In this case, functional features of the method are embodied by objective units or elements of the apparatus, for instance.

According to a first aspect, a computer-implemented method for sharing medical data sets comprising several steps is provided. A first step is directed to receiving medical data sets from a medical system. A second step is directed to receiving associated information from a database separated from the medical system, wherein the associated information provides information associated to the medical data sets. A further step is directed to uploading the medical data sets to a cloud platform. A further step is directed to processing the associated information and the medical data sets to identify one or more entitled users, the entitled users being entitled to access a respective medical data set. Yet, a further step is directed to retrieving additional user information of the entitled users, the additional user information being different from the associated information. A further step is directed to granting the entitled users access to the respective medical data set in the cloud platform on the basis of the additional user information.

In other words, it is an idea of the present invention to integrate at least two different data sources (i.e., the medical data sets and the associated information) and combining them with cloud technology for arriving at an automized computer-implemented workflow for sharing medical data sets with entitled users.

The medical data sets can be conceived as generally relating to test results of a patient. For instance, these test results may include medical image data sets comprising medical images acquired using a medical imaging modality. A medical imaging modality corresponds to a system used to generate or produce medical images. For example, a medical imaging modality may be a computed tomography system, a magnetic resonance system, an X-ray system, an angiography (or C-arm X-ray) system, a positron-emission tomography system or the like. In addition, medical images may be generated by digitally recording microscope image of tissue slices. Correspondingly, a medical image may be a computed tomography image, a magnetic resonance image, an X-ray image, an angiography image, a positron-emission tomography image, digital pathology image or the like. The medical image data may be a two-, three or four-dimensional medical image, either providing two and/or three dimensions in space, with or without an additional dimension in time. Besides or in addition to medical images, the medical data sets may relate to other examination results of a patient such as laboratory data, or to other personal test data of the patient, for instance.

The medical system may generally be configured to acquire and/or forward and/or store medical data sets. For instance, the medical system may be embodied as a medical image system comprising one or more medical imaging modalities and/or one or more data storage and archiving system for medical image data. The medical image system may comprise a picture archiving and communication systems (PACS). As yet another example, the medical system may comprise equipment for acquiring laboratory test data of patients and/or facilities for storing and archiving the corresponding medical data sets. Further, the medical system may comprise any combination of the aforesaid.

The associated information relates to data separate from or outside of the medical data sets and/or to data not contained in the medical data sets. The associated information contains information associated with a respective medical data set in the sense that the associated information is additional or supplementary information complementing or corresponding to the respective medical data sets. The associated information may be information which was not recorded in the medical data set or for which there is no designated variable in the medical data set at all. As such, the associated information may be administrative information pertaining to medical, administrative, financial, and legal issues. Moreover, the associated information may contain the patient's health record, information about workflows within an organization, information relating to the outside of the organization such as personal information of a referring physician, or structured or unstructured diagnosis reports. The associated information may be provided in the form of an electronic medical record (EMR), other electronic health records, electronic clinical pathways, patient identification records, diagnosis reports, similar patient studies, electronic laboratory reports or the like.

The association of the associated information to the medical data sets can be conceived as a structural association electronically linking these two data types. In other words, the medical data sets and the associated information may be interconnected via reference tags in the form of unique identifiers so that each medical data set is unambiguously related to the corresponding associated information. The structural association may be embodied by a unique identifier such as an electronic data identifier or any other suitable electronic tag. Further, the unique identifier may comprise the patient's ID or an accession number. The unique identifier can also comprise a hash of the data mentioned before and/or any combination thereof. The unique identifier serves as a link between the medical data sets and the associated information. According to an embodiment, all medical data sets and associated information created within the clinical environment for a specific patient bear the same unique identifier. Accordingly, an association between a given medical data set and a given associated information can be established based on a comparison or matching the unique identifier (i.e., the respective electronic data identifiers or tags). In this regard, an association may be determined, if the respective electronic data identifiers or tags correspond, match or are identical.

The database may generally be seen as being configured to generate and/or store and/or forward the associated information. The database may relate to medical information systems such as the HIS, RIS, LIS, CIS or any other information system for archiving and exchanging data and complementary information associated to medical data.

The database being "separate" from the medical system may mean that the database relies on a different data format or data standard for storing and transmitting the associated information than the medical system for storing and transmitting the medical data sets and/or that the database is physically sperated from the medical system. The latter may be embodied by different and/or independent local or spread storage systems and/or server systems for the database and the medical system, for instance. "Being separate" may further mean that neither the medical system has direct access to the database nor that the database has direct access to the medical system. "No direct access" in this respect may mean that the database and the medical system cannot directly exchange information or actively query each other for retrieving information. In a way, the above method may thus be conceived as a computer-implemented connector or intermediary between the database and the medical system.

In other words, the steps of receiving (either the medical data or the associated information) may be equivalent to automatically receiving corresponding messages from the respective sources (either the medical system or the database) or listening to the respective data streams from these sources by any computer system embodying the method. Moreover, the steps of receiving may optionally be preceded by actively querying the respective sources (the medical system and/or the database) for the corresponding data (medical data sets and/or associated information).

The medical system and the database may be seen as representing the local architecture inside a given local environment such as a clinic consortium, hospital, practice or imaging center. The cloud platform may, e.g., relate to a central cloud-server outside of and remote from the local environment. The cloud platform may be accessible for authorized web services via cloud storage gateways and may comprise a real or virtual group of computers remote from the local environment. Alternatively, the cloud platform may be embodied as an externally accessible local exchange server within the local architecture of the given organization.

In the step of processing the associated information and the medical data sets, the respective messages may be automatically evaluated. The subsequent identification of one or more entitled users may comprise searching the received data packages for a hint or an indication of one or more entitled users. Such signature can comprise an explicit reference to the entitled user contained in the data. In addition to that or as an alternative, metadata such as headers or data tags of the medical data sets and the associated information may be searched. Likewise, data indicators or electronic tags representative of the owner of the respective data, the patient concerned, or any other entitled person may be evaluated to identify the entitled user. The data indicator can be a personnel number, a patient ID or an account number, for instance. Electronic tags can be abstract electronic data identifiers indicative of the entitled user.

For granting the entitled users access to the medical data sets (and thus sharing the medical data sets), more is need than the mere finding who the entitled user actually is. To start with, an entitled user has to be informed that medical data sets have been uploaded to the cloud platform for him. Further, it is to be established how the user may get access to the cloud platform. For instance, some users have a permanent account for the cloud platform while others don't. Finally, means and information must be provided and forwarded to the user so that he can access the respective medical data set. For that reason, the method relies on additional user information which is different and additional to the mere identification of the entitled user. Therefore, once the entitled user or the group of entitled users is identified, secondary information pertaining to the user (= additional user information) is automatically retrieved. This information is then used to automatically grant the identified entitled users access to the medical data uploaded to the cloud platform. In contrast to medical data sets and associated information, the additional user information does relate to (or does not include any) medical information. Rather, it may include personal information about the entitled users such as name, social security number, birth date, address, telephone numbers and the like. Specifically, it may comprise information about the ways, an entitled user may get access to the cloud platform such as whether or not the entitled user has a user account with the cloud platform.

Taking all this together, the outlined method steps according to the first aspect synergistically contribute to an automated data exchange of medical data between entitled users that seamlessly integrates into existing clinical workflows. In particular, the method allows for the selective retrieval of all the pieces of information needed for sharing medical data sets with entitled users. By integrating multiple separate data sources, the method automatically copes with any inconsistencies in the data records. For instance, since all of the available information is integrated and processed for identifying the entitled users, the method works no matter in which of the available data sources (i.e., medical system or database) or in which instances of the clinical workflows the entitled users have been recorded. Likewise, the autonomous retrieval of the additional user information required for sharing the medical data sets enables the method to flexibly adapt to various different workflows in the clinical environment. At the same time, the automatic processing and retrieval of information makes the method according to the first aspect highly fail-safe and secure. Further, by including an automated upload of the medical data into a cloud platform, the method enables a convenient access to the medical data for the entitled users. What is more, since the process of sharing is carried out via the cloud platform, the healthcare organizations do not have to grant access to their local databases as such, which contributes to an enhanced data security.

For the step of granting access, the additional user information and/or an indication of the entitled users for each uploaded medical data set may be forwarded to the cloud platform. The step of granting access may then be (predominately) performed at the cloud platform. Alternatively, the cloud platform may further be configured to retrieve the additional user information on its own based on an indication of the one or more entitled users (which is then forwarded to the cloud platform). As yet a further alternative, the step of granting access may be predominantly performed locally (i.e., not at the cloud platform) wherein necessary steps at the cloud platform are triggered, commanded and controlled locally by an appropriate system such as a local connectivity node or the like.

According to an embodiment, the medical data sets may be formatted according to a first communication standard (or, in other words, may be formatted in a first standardized format) and the associated information may be formatted according to a second communication standard (or, in other words, may be formatted in a second standardized format), wherein the first communication standard is different from the second communication standard (wherein, in other words, the first standardized format is different from the second standardized format). Standardized formats or communication standards may relate to proprietary formats or open standards used for administrating medical data sets and/or associated information.

By taking different standards (communication standards, standardized formats) into account, the method can be seamlessly integrated into existing clinical workflows which often rely on different standards for medical data sets and the corresponding associated information.

According to an embodiment, the medical data sets may be formatted in the DICOM format (as the first communication standard). Further, the step of receiving medical data can comprise receiving the medical data by using a dedicated DICOM Application Entity Title (DICOM AET), which may be done by assigning a DICOM AET, e.g., to an interface for receiving the medical data sets or any other device involved in putting the method steps into practice.

DICOM (=Digital Imaging and Communications in Medicine) is an open standard for the communication and management of medical imaging information and related data in healthcare informatics. DICOM may be used for storing and transmitting medical images enabling the integration of medical imaging devices such as scanners, servers, workstations, printers, network hardware, and picture archiving and communication systems (PACS). It is widely adopted by clinical syndicates, hospitals, as well as for smaller applications like doctors' offices or practices. A DICOM data object consists of a number of attributes, including items such as patient's name, ID, etc., and also one special attribute containing the image pixel data. A single DICOM object can have only one attribute containing pixel data. For many modalities, this corresponds to a single image. However, the attribute may contain multiple "frames", allowing storage of cine loops or other multiframe data.

However, DICOM not only defines a standard for data objects but a whole communication standard clamping down on the design of hardware components. DICOM files can be exchanged between two entities that are capable of receiving and/or processing image and patient data in DICOM format. As such, they are, in other words, compatible to the DICOM format or represent so-called DICOM-nodes. In the framework of the present invention, the medical system as well as the system for sharing medical data sets may be compatible with the DICOM format or may be DICOM nodes.

DICOM AE Title or AET is an abbreviation for Application Entity Title. An AE Title is used by an Application Entity (AE) (i.e., a DICOM node or DICOM compatible device) to identify itself in a network. DICOM AETs are locally unique and are typically managed by a system administrator. Thus, by relying on the DICOM AET, the communication according to the method can be streamlined, since the medical data sets can be automatically forwarded to the computer-implemented method for further processing.

According to an embodiment, the associated information may be formatted in the HL7 and/or FHIR standard (as the second communication standard).

HL7 (Health Level Seven) specifies a set of flexible standards, guidelines, and methodologies by which various devices (such as databases and/or workstations) in healthcare informatics can communicate with each other. It is different from the DICOM standard as regards the underlying technology as well as the field of application. While DICOM focusses on medical image data sets, HL7 is predominately used for administration, e.g., as regards electronic patient records. Like DICOM, HL7 allows information to be shared and processed in a uniform and consistent manner and therefore enables to easily share clinical (medical) information. The FHIR (Fast Healthcare Interoperability Resources)-standard builds on previous standards from HL7 and uses a web-based suite of API-technology. It is meant to enhance the interoperability and support a wider variety of devices from workstations to tablets to smart phones.

By relying on either the DICOM and/or the HL7 and/or the FHIR standard, the method is compatible with the commonly used formats and can be readily implemented in existing clinical workflows. Thereby, the assignment of a DICOM AET has the particular benefit that the medical data sets can be automatically routed for further processing.

According to an embodiment, in the step of retrieving, the additional user information is extracted from the associated information, and/or the additional user information is retrieved from the database, and/or the additional user information is retrieved from a separate user data repository. In other words, the step of retrieving may comprise querying different data sources for additional user information of the respective entitled users.

By taking into account these sources for retrieving the additional user information, the method can ensure that the additional user information required for sharing the medical image data with the entitled user is actually available. This is because the additional user information required for sharing the medical data sets with the entitled users is typically spread within the healthcare organization. One source could be the medical data sets themselves. However, since the medical data sets are usually recorded prior to (or even long before) sharing the data, the medical data sets are typically deficient in additional user information other than the mere indication of a corresponding patient (via, e.g., the accession number or patient ID). Thus, it is more likely to find the additional user information in the associated information or the corresponding database since these sources of information pertain to the administration of the healthcare organization. In addition to that, separate user data repositories may be considered. Such user data repositories can be embodied by dedicated cloud address books, for instance. The user data repositories may be separate from the medical system and/or the database.

According to an embodiment, the additional user information comprises the entitled users' email address, the entitled users' phone number, the entitled users' account information with respect to the cloud platform, the entitled users' address and/or any combination thereof.

The additional user information may be used by the method to figure out how a specific entitled user can get access to the medical data set (e.g., via an existing account). Likewise, address and contact information may be used to automatically contact the user and inform him that medical data sets have been uploaded to the cloud platform for him and communicate the specific procedure for getting access to the medical data sets. In this respect, the additional user information is functional data that is used to control the computer implemented method for sharing medical data sets. Accordingly, the additional user information inherently reflects features of the method (and the claimed system).

According to an embodiment, the step of granting access may comprise the step of establishing whether or not the entitled user has an account in the cloud platform on the basis of the additional user information.

This has the benefit that the method can flexibly react to the respective requirements on the part of the entitled users. While it can be assumed that professional healthcare providers such as referring physicians/specialists or other hospitals have a permanent user account in the cloud platform, this will usually not be the case for patients or the family doctor. Accordingly, the method automatically takes into account different ways for sharing medical data sets with different entitled users and is therefore more versatile when it comes to integrate it into existing workflows.

If the entitled user has a user account in the cloud platform, the method may optionally proceed with the step of mapping the entitled user to his user account in the cloud platform on the basis of the additional user information. If not, the method may optionally proceed with the steps of respectively creating unique passcodes for the entitled users and/or respectively generating URLs for the entitled users with which the entitled users can access the respective medical data sets in the cloud platform and forwarding the URL to the entitled user.

According to an embodiment the step of granting access comprises mapping (or, in other words, assigning) the entitled users to corresponding user accounts of the entitled users in the cloud platform on the basis of the additional user information, and making the medical data sets accessible to the entitled users via the account.

The mapping of the additional user information to a user account provides a convenient way for accessing the shared data, as it builds on existing infrastructure. In this regard, the step of making the medical data sets accessible may comprise providing an electronic link to the respective medical data sets in the user account with which the entitled user can access or download the medical data sets when logging on to his user account. Moreover, this step further contributes to the data security of the method since a permanent account is usually assigned to users that have been duly accredited.

Optionally, the method may comprise the step of communicating, to the entitled users, that a medical data set has been assigned to their corresponding user accounts, e.g., via an email or SMS (acronym for "Short Message Service") or the like. This increases the user-friendliness of the method.

According to an embodiment, the step of granting access may comprise respectively generating URLs for the entitled users, with which the entitled users are enabled to access the respective medical data sets in the cloud platform, and forwarding the URLs to the entitled users on the basis of the additional user information.

By providing entitled users with URLs to access the medical data sets, it is possible to address those entitled users lacking a permanent account to the cloud platform - as may typically be the case for patients or smaller practices. In doing so, the method automatically addresses different ways for accessing the cloud platform and further eliminates manual interventions when routing information to the respective recipient. In particular, there is no need to register the entitled user in the cloud platform, which may be cumbersome for one-time access.

According to an embodiment, the step of granting access may comprise respectively creating unique passcodes for the entitled users, respectively forwarding the unique passcodes to the entitled users on the basis of the additional user information, and respectively granting access to the medical data sets upon receipt of the unique passcodes from the entitled users.

The provision of unique passcodes further contributes to the data security of the method as this ensures that only entitled users get access to the medical data sets.

The steps of creating and forwarding unique passcodes and providing access upon receipt of the unique passcodes from the entitled users may be combined with the aforementioned steps of granting access via the user accounts or via the provision of URLs. For both cases, the provision of the unique passcodes further increases the data security.

As a further alternative in this regard, the method may comprise using different communication channels for forwarding the URLs to the entitled user or notifying the entitled user that data has been mapped to his user account on the one hand and for forwarding the passcodes on the other hand. The different information channels may, for instance, comprise email or SMS messages, phone calls, messenger services, messages using web clients, or even postal messages and printouts. The corresponding contact data and preferred ways of contacting can be retrieved from the additional user information.

According to an embodiment, the method may further comprise extracting unique identifiers from the medical data sets received and associating the corresponding associated information to the medical data sets on the basis of the unique identifiers.

Thus, the unique identifiers are used to identify the associated information for the respective medical data set, or, in other words, to match the respective medical data set to the corresponding associated information.

In that sense, a given medical data set may match the associated information if they have the same unique identifier. Accordingly, the step of associating can comprise extracting second unique identifiers from the associated information and associating the associated information to the corresponding medical data sets if the unique identifiers and the second unique identifiers correspond.

In other words, the process of matching a given medical data set with the associated information (associating the associated information to the medical data set) may comprise identifying all available associated information having the same unique identifier as the medical data set in the data stream coming from the database and associating this information to the medical data set.

The use of unique identifiers is beneficial for swiftly identifying the correct associated information to a given medical data set. Accordingly, this improves the integratability of the method into existing workflows, makes it readily scalable and ensures that all relevant entitled users are identified.

In practice, unique identifiers are often provided for by an accession number assigned to each new patient entering a healthcare environment or a patient ID. As mentioned, the unique identifier may be any suitable electronic data identifier or electronic tag, however.

According to an embodiment, the method may comprise the step of determining whether or not the received medical data sets are to be shared with all or part of the entitled users. The step of determining may be based on the medical data set, the associated information and/or the additional user information. Further the step of determining may comprise reading the medical image data sets, the associated information and/or the additional user information. In this regard, the method may read metadata as well as the body of the medical data sets, the associated information and/or the additional user information. Whether or not a received medical data set is to be shared with all or part of the entitled users may be encoded in the medical data sets, the associated information and/or the additional user information in the form of electronic identifiers or tags, data flags or text.

Optionally, if it is determined on that basis that the received medical data sets are to be shared with the entitled users, the method uploads the medical image data to the cloud platform. If not, the method refrains from doing so.

This has the advantage that only such data is shared/uploaded to the cloud platform which actually shall be shared. This limits data traffic and at the same time further improves the data security.

According to an embodiment, the step of processing comprises accessing and reading the associated information to identify one or more entitled users from the associated information and accessing and reading the medical data to identify one or more entitled users from the medical data set.

The accessing and reading of the available data sets and information ensures that all entitled users are correctly identified no matter in which data base the respective entitlement has been recorded. This fosters the integrability of the method into existing workflows.

According to an embodiment, the step of processing may comprise reading metadata of the received data packages (i.e., medical data sets or associated information), wherein the metadata may comprise headers and/or specific electronic tags or identifiers indicating the entitled users.

With the processing and reading of metadata for identifying the entitled users, the method takes advantage of the fact that the data packages used in a medical environment are standardized and often comprise default metadata pertaining to the identity of the patient and/or the referring physician or other entitled users (with the DICOM standard being a prime example in this regard). This has the advantage that a comparatively fast data processing ensues. Identifying attributes available as metadata may, for instance, be patient names, patient IDs, accession numbers, birthdates, sex, and the like.

In addition to that or as an alternative, the processing step may as well comprise reading the body or all of the received data packages (i.e., medical data sets and associated information), especially if the data packages received are found not to be provided in a standardized format, as may often be the case for patients' reports. Further, additionally or alternatively, the processing step may comprise reading text from the medical data sets, the associated information and/or the additional user information. To this end, the method may rely on a semantic search tool or text recognition algorithms such as OCR (acronym for "optical character recognition"). This has the advantage that all relevant information can be accessed for sharing the medical data sets.

According to an embodiment, the method may further comprise the step of querying the database for the associated information. Optionally, this step may be carried out upon receipt of the medical data sets or may be triggered by receiving the medical data sets.

By actively querying the database for the associated information, the method can make sure to promptly retrieve all the information required for identifying the entitled users.

Likewise, the process of querying the database for associated information can comprise determining a unique identifier from the medical data set and querying the associated information using the unique identifier. The unique identifier may be a patient ID or an accession number or any other suitable electronic identifier uniquely identifying a patients' case or clinical process.

The use of unique identifiers is beneficial for swiftly querying the database for the correct associated information corresponding to a given medical data set. Accordingly, this improves the integration of the method into existing workflows and ensures that all relevant entitled users are identified.

According to an embodiment, the entitled users may comprise patients, referring physicians, close relatives, parents or legal guardians or health insurance personnel and/or referring hospitals.

By addressing this group of entitled users, the method serves the main recipients for sharing medical data sets and provides a unifying solution for several different requirements - what makes the method cost-effective and user-friendly.

According to another aspect, an alternative computer-implemented method with numerous steps is provided. A first step is directed to receiving medical data sets from a medical system. A further step is directed to uploading the medical data sets to a cloud platform. A further step is directed to querying a database separate from the medical system for associated information, wherein the associated information provides supplementary information associated to the medical data sets. A further step is directed to processing the associated information and/or the medical data sets to identify one or more entitled users, the entitled users being entitled to access a respective medical data set. A further step is directed to retrieving additional user information of the entitled users. A further step is directed to granting the entitled users access to the respective medical data set in the cloud platform on the basis of the additional user information.

According to another aspect, a system for sharing medical data sets is provided. The system comprises an interface unit configured to communicate with a medical system for receiving medical data sets, with a database for receiving associated information, and with a cloud platform for uploading the medical data sets. Thereby, the database is separate from the medical system and the associated information provides information associated to the medical data sets. Further, the system comprises a computing unit, which is configured to receive medical data sets and associated information from the interface unit, to identify one or more entitled users on the basis of processing the medical data sets and the associated information, to retrieve additional user information of the entitled users, to upload the medical data sets to the cloud platform via the interface unit, and to grant the entitled users access to the respective medical data set in the cloud platform on the basis of the additional user information. Thereby, the entitled users are entitled to access respective medical data set.

The system may be conceived as a connectivity node or routing system, which integrates and processes different data sources to automatically relay the medical data sets received to the entitled users for sharing.

The computing unit can be realized as a data processing system or as a part of a data processing system. Such a data processing system may, for example, comprise a cloud-computing system, a computer network, a computer, a tablet computer, a workstation and/or the like. The computing unit can comprise hardware and/or software. The hardware can be, for example, a processor, a memory and combinations thereof. The hardware can be configurable by the software and/or be operable by the software. Generally, all of these potential units, sub-units or modules of the computing unit may be at least temporarily be in data exchange with each other, e.g. via network connection or respective interfaces. Consequently, individual units may be located apart from each other.

The interface unit may be configured to communicate over one or more connections or buses. The interface unit may be embodied in the form separate physical interfaces each providing a separate connection to the respective databases, medical systems or repositories and optionally relying on different network standards such as (Ethernet, DSL, PPPoE, ISDN, ATM). Alternatively, the interface unit may be embodied as a (unique) gateway or interface to a network (such as a Ethernet port or WLAN interface) via which all necessary communication is effected.

According to an embodiment, the system is assigned a specific destination address so that the medical image data may be automatically routed to the system. To this end, a DICOM AET may be assigned to the system, for instance (or, in other words, the system may comprise a DICOM AET).

This has the effect that medical data sets can be automatically routed to the system for further processing and, eventually, sharing with the entitled users.

According to an embodiment, the computation unit is further configured to retrieve the additional user information from the associated information.

According to an embodiment, the computation unit is further configured to retrieve the additional user information from a user data repository via the interface unit, with the interface unit being further configured to communicate with the user data repository for retrieving additional user information. The user data repositories may be separate from the other data sources (i.e., medical system and database).

According to another aspect, an environment for sharing medical data sets is provided which comprises the system for sharing medical data sets as introduced above and the medical system, wherein the medical system is configured to acquire and/or store and/or exchange the medical data sets.

With that, an integrated system is provided which complements the functionality of the medical system with an automatic procedure for sharing the medical data sets. This allows for a swift integration into existing clinical workflows.

According to an embodiment, the environment for sharing medical data sets further comprises the database and/or the user data repository.

According to another aspect, the present invention is directed to a computer program product comprising program elements which induce a computing unit of a system for sharing clinical data sets to perform steps according to the inventive method, when the program elements are loaded into a memory of the computing unit.

According to another aspect, the present invention is directed to a computer-readable medium, on which program elements are stored that are readable and executable by a computing unit of a system for sharing clinical data sets in order to perform steps of the inventive method when the program elements are executed by the computing unit.

The realization of the invention by a computer program product and/or a computer-readable medium has the advantage that already existing systems can be easily adopted by software updates in order to work as proposed by the invention.

The computer program product can be, for example, a computer program or comprise another element next to the computer program as such. This other element can be hardware, for example, a memory device, on which the computer program is stored, a hardware key for using the computer program and the like, and/or software, for example, a documentation or a software key for using the computer program. The computer program product may further comprise development material, a runtime system and/or databases or libraries. The computer program product may be distributed among several computer instances.

Characteristics, features and advantages of the above described invention, as well as the manner they are achieved, become clearer and more understandable in the light of the following description and embodiments, which will be described in detail with respect to the figures. This following description does not limit the invention on the contained embodiments. Same components or parts can be labeled with the same reference signs in different figures. In general, the figures are not to scale. In the following:
Figure 1 depicts systems for sharing medical data sets according to embodiments, and
Figure 2 depicts a method for sharing medical data sets according to embodiments.

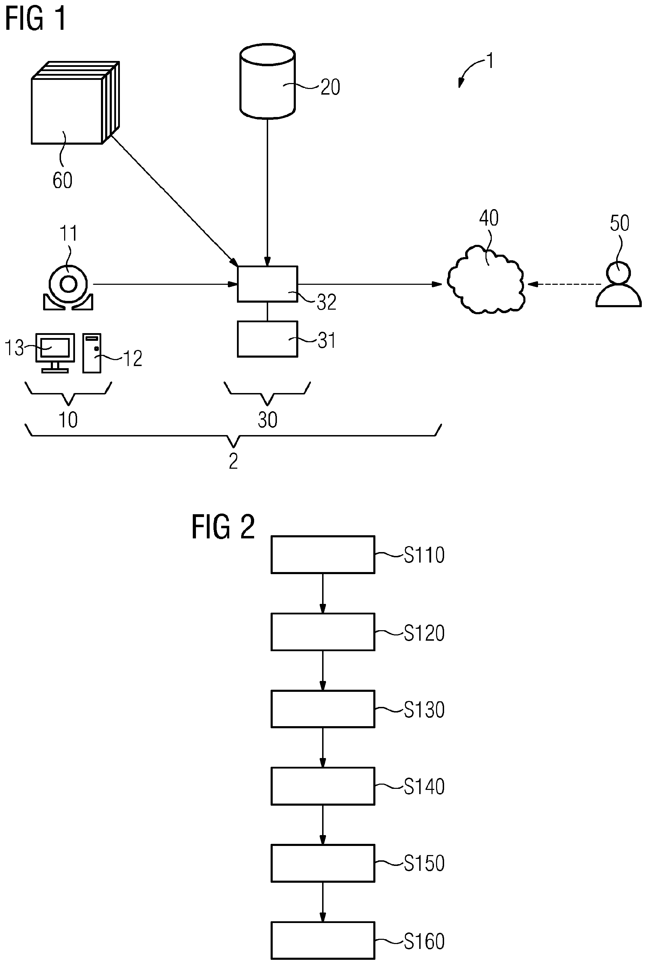

Figure 1 depicts a system architecture 1 for sharing medical data sets according to an embodiment. The system architecture 1, or parts of the system architecture 1, are configured to perform the methods according to one or more embodiments of the invention, e.g., as described with reference to Figure 2.

The system architecture 1 according to one or more embodiments may comprise a medical system 10, at least one database 20, a system for sharing medical data sets 30 (in the following referred to as "connectivity node"), a cloud platform 40, and, optionally, at least one user data repository 60. "System for sharing medical data sets" as used in this context is not be construed as exclusively relating to device 30. Rather, the "system for sharing medical data sets" as used throughout the present application may encompass further component parts or devices of the system architecture 1 such as the medical system 10 and/or the database 20 and/or the cloud platform 40.

The system architecture 1 comprises a part 2 (subsequently also designated as "local environment 2") which may be predominately locally installed in a clinical or medical environment such as a clinical consortium (i.e., an association of two or more hospitals), hospital, imaging center or radiology practice, for instance. Other parts of the system architecture 1 may be remote from the local components, such as the cloud platform 40.

In the example shown in Figure 1, the medical system 10, the database 20, the connectivity node 30 and the user data repository 60 are implemented locally in the local environment 2. The cloud platform 40 is remote from the local environment 2. This is not be construed as limiting the disclosure of the present invention, however. For instance, components like the database 20 or the user data repository 60 may likewise be sourced out from the local environment 2 to remote devices. In turn, cloud platform 40 may be installed locally at the local environment 2 as well.

Individual components of the system architecture 1 may be at least temporarily connected to each other for data transfer and/or exchange. Medical system 10 communicates with connectivity node 30 via its interface unit 32 to transfer medical data sets. For example, connectivity node 30 may be activated on a request-base, wherein the request is sent by medical system 10. Connectivity node 30 further communicates with database 20 and user data repository 60 via interface unit 32. Database 20 and repository 60 may likewise be activated on a request-base, wherein the request is sent by connectivity node 30.

Data transfer is preferably realized using a network connection. The network may be realized as local area network (LAN), e.g., an intranet, ethernet or a wide area network (WAN), e.g., the internet. Network connection is preferably wireless, e.g., as wireless LAN (WLAN or Wi-Fi). The network may comprise a combination of the different network types.

The medical system 10 is generally configured to acquire and/or store and/or archive and/or forward medical data sets of a patient. The medical data sets are unambiguously related to a patient, e.g., by providing each medical data set with a unique identifier indicative of the respective patient. Medical system 10 may be configured such that it cannot be accessed from the outside of the local environment 2. This may include that it is configured such that it cannot be (directly) accessed by the entitled users 50.

For each medical data set, there usually exists at least one entitled user 50 who is entitled to get access the medical data set. Typically, this is the patient himself and/or his attending physician. In many cases, medical data sets have more entitled users 50 than that, however. Other entitled users 50 may be referring or consulting physicians, specialists such radiologists, close relatives, parents or legal guardians or health insurance personnel. In most cases, not all of these entitled users 50 are recorded in the medical data sets, however.

Medical system 10 as depicted in Figure 1 is configured as a medical imaging system. It comprises a medical imaging modality 11 for acquiring medical image data, such as a computed tomography system or a magnetic resonance system, an angiography (or C-arm X-ray) system, a positron-emission tomography system or the like. Moreover, medical system 10 may comprise an archive/review station 12 for storing, archiving and reviewing medical data such as a PACS. Storing comprises incidental temporal storage of data and permanent storage in the sense of archiving.

Additionally, medical system 10 may comprise user interfaces 13 for reviewing, processing and/or forwarding medical data sets. In addition to that, the medical image system 10 may comprise routers or nodes for forwarding medical image data (not shown).

Alternative embodiments for medical system 10 may comprise multiple imaging modalities 11 and multiple archive/review stations 12. To interconnect the various components, the medical system 10 may comprise a network (not shown). In accordance with an embodiment, the network comprises a DICOM compatible network. DICOM is a network protocol that sits on top of TCP/IP. Messages according to the DICOM protocol include a header with metadata (for instance, comprising patient information) and information in the form of an image. DICOM allows interoperability between the various medical systems and is able to exchange both image data and reports between the systems. In accordance with an embodiment, the imaging modalities 11, the archive/review stations 12 as well as any further nodes in medical system 10 are DICOM compatible devices, making the medical system 10 as such a DICOM compatible device.

The network connecting medical system 10 to the connectivity node 30 may likewise comprise a DICOM compatible network. Accordingly, connectivity node 30 may as well be configured as DICOM compatible device in the form of, for instance, a DICOM node or DICOM Application Entity having a DICOM Application Entity Title.

Alternative embodiments of the present invention may comprise multiple networks using different network protocols which may be the same as or different than DICOM. In that case, the DICOM Application Entity Title would be any other suitable identifier for identifying connectivity node 30 in the network.

In the foregoing, the medical system 10 has been described as a medical image system configured to acquire and/or archive and/or forward medical image data. However, this is to be construed by ways of example and not as limitation. The medical data sets may relate to medical image data, laboratory data of a patient, patient records, diagnosis reports, clinical studies or the like and combinations thereof. Correspondingly, the components of the medical system 10 may be configured to acquire and/or archive and/or forward this data.

Database 20 is configured for generating and/or storing and/or exchanging associated information which is associated to the medical data sets of a patient. According to an embodiment, the database 20 is part of a hospital information systems (HIS), radiology information systems (RIS), clinical information systems (CIS), laboratory information systems (LIS) and/or cardiovascular information systems (CVIS). Database 20 may be configured such that it cannot be accessed from the outside of the local environment 2. This may include that it is configured such that it cannot be (directly) accessed by the entitled users 50. The associated information administrated by the database 20 may relate to supplementary administrative information corresponding to a respective patient. As an alternative or in addition to that, the associated information may pertain to a health record or heath history of the respective patient. As such, the associated information in one way or the other may provide indications about entitled users 50 for the respective case.

In addition to that, the associated information may contain additional user data such as contact information for the entitled users 50 mentioned in the associated information.

Like the medical data sets, the associated information is unambiguously related to a patient. Electronically, this may be provided for by a unique identifier which is preferably the same as used for the medical data sets. For instance, such a unique identifier may be embodied by the accession number of the patient and/or case or a patient ID, e.g., the social insurance number of the patient.

Database 20 may be realized as a cloud storage. Alternatively, database 20 may be realized as a local or spread storage. Database 20 may comprise a plurality of individual repositories as well as user interfaces (not shown) for generating, reviewing and/or processing associated information such as workstations for generating diagnosis reports or administratig an electronic medical record of a patient. Like medical system 10, database 20 may comprise a network compatible to a particular standard for interconnecting the individual components of database 20 and facilitating data exchange. According to an embodiment, this network may be a network compatible with the HL7 and/or FHIR standards.

Optionally, the local environment 2 may comprise a further user data repository 60 for storing additional user information for part or all users registered in the environment. Database 20 and repository 60 may likewise be combined in one database comprising (archiving) both the associated information as well as the additional user data. According to an embodiment, repository 60 may be conceived as an electronic address book. In contrast to the associated data, there is no unambiguous relation between the additional user information and the medical data sets by means of a fixed association in the form of a dedicated electronic identifier or the like linking the additional user information to a specific case. Additional user information as stored in repository 60 can typically only retrieved on the basis of the username. User data repository 60 may be realized as a cloud storage. Alternatively, user data repository 60 may be realized as a local or spread storage. Moreover, it is possible that the user data repository 60 resides outside of the local environment 2 and is provided in the form of an external cloud service, for instance.

Database 20 and user data repository 60 may be updated continuously and/or on a daily or weekly basis. In parallel, database 20 may be updated with new entries as soon as a workflow within the local environment (such as a patient examination within a hospital) or the like is completed.

The network connecting the database 20 and the connectivity node 30 may comprise a HL7 and/or FHIR compatible network. As an alternative or in addition to that, the network may support any other communication standard for exchanging data between connectivity node 30 and database 20. The same applies for the network connecting connectivity node 30 and user data repository 60.

Connectivity node 30 may comprise a processor 31 and an interface unit 32 for data exchange. Interface unit 32 may comprise individual interfaces configured to communicate with individual components of the system architecture 1. Alternatively, interface unit 32 may be configured as uniform interface configured for data exchange with all of the components of the system architecture 1. Interfaces for data exchange may be realized as hardware- or software-interface, e.g., a PCI-bus, USB, ethernet-connection or firewire.

Processor 31 may comprise a hardware or software component, e.g., a microprocessor or a FPGA ('Field Programmable Gate Array). Processor 31 is configured to perform steps according to the methods as explained in connection with Figure 2.

Could platform 40 may be a central cloud-server which is accessible for authorized web services for entitled users 50. Access may be granted via dedicated user accounts or via one-time sign-ins. Cloud platform 40 may comprise a real or virtual group of computers. Cloud platform 40 may be located outside of the local environment 2 as shown in Figure 1. Alternatively, cloud platform 40 likewise may be embodied as an externally accessible local exchange server within the local environment 2, however.

While the network components connecting connectivity node 30 with the other (preferably locally installed) system components (such as medical system 10 or database 20) are usually bi-directional and able to communicate in both directions between interconnected components, the network connection to cloud platform 40 may be single directional in the sense that uploading data to cloud platform 40 is possible but access from cloud platform 40 to the other components of the local environment 2, e.g., via connectivity node 30 is limited, denied and/or right away not possible. Of note, system architecture 1 may be configured such that cloud platform 40 is only connected to connectivity node 30 an no other components of the local environment 2.

The inventive computing unit for sharing medical data sets may comprise processor 31 of connectivity node 30. Accordingly, at least parts of the inventive method may run on processor 31 of connectivity node 30. Further, the computing unit may comprise additional sub-units (not shown), spread in the system architecture 1. Such sub-units may, for instance reside in the medical system 10, the database 20, the user data repository 60 or other components of the local environment 2 in the form of micro-controllers, integrated circuit or real or virtual group of computers like a so called 'clusters' or 'clouds'. Moreover, sub-units of the computing unit may also reside in components outside of the local environment 2 such as in the cloud platform 40.

Analogously, the inventive interface unit may comprise interface unit 32 of connectivity node 30, but also other interfaces of the other components linked thereto, such as interfaces at medical system 10, database 20, user data repository 60 or cloud platform 40.

According to one embodiment, the inventive system may thus be embodied by connectivity node 30 alone comprising the interface unit 32 and the processor 31 of connectivity node 30. However, the system may further encompass additional components such as parts or all of medical system 10, database 20, user data repository 60 or cloud platform 40 according to other embodiments of the invention.

Figure 2 is a flowchart depicting a method for sharing medical data sets according to an embodiment. The method comprises several steps. The order of the steps does not necessarily correspond to the numbering of the steps and/or the depicted sequence of steps but may also vary between different embodiments of the present invention.

A first step S110 is directed to receiving, by connectivity node 30, a medical data set. The medical data set may be sent to connectivity node 30 automatically or manually by a user such as a radiologist or technician. For that purpose, connectivity node 30 may be provided with suitable network identifier, such as a network address, or, in the case of a DICOM compatible network, a DICOM Application Entity Title. In other words, connectivity node 30 is configured to listen to the data stream from medical system 10 and to receive medical data sets being sent to its address in the network.

Any medical data set relayed to connectivity node 30 will be automatically further processed by the subsequent steps of S120 to S160.

For sharing the medical data sets with entitled users 50, connectivity node 30 may be configured to upload the medical data sets to cloud platform 40 in step S120. Optionally, step S120 may comprise a step of determining, on the basis of the oncoming medial data sets, whether or not a specific medical data set is to be shared with entitled users 50 in the first place. This optional step is preferably executed prior to uploading the respective medical data set to cloud platform 40. To this end, the medical data sets may be read and checked for a suitable electronic identifier ("sharing flag") indicating that a respective medical data set is to be shared. If this is the case, the method proceeds with uploading the medical data sets to the cloud platform 40 according to step S120. Such sharing flag may, for instance, be set by a user (e.g., radiologist or technician) and may be encoded in the header of the medical data sets.

In parallel to listening to the data stream from medical system 10, connectivity node 30 is configured to listen to the data stream from database 20 for receiving information associated to respective medical data sets (step S130). The associated information received may then be matched to the respective medical data set. For instance, this can be done by reading from the associated information and the medical data sets unique electronic identifiers indicative of the particular case or patient, such as a patient ID or accession number, and comparing the unique identifiers. Optionally, step S130 may comprise a step of actively querying database 20 for associated information corresponding to a respective medical data set. This optional step may likewise be carried out using the unique identifiers, e.g., by reading from the respective medical data set one or more unique identifier and querying database 20 for associated information having the same unique identifier. For reading the unique identifier, connectivity node 30 may be configured to look at the headers of the medical data sets or the associated information in step S130.

The aforementioned optional assessment of whether or not a medical data set is to be shared with entitled users 50 may likewise also be comprised in step S130. To this end, the associated information may be read and checked for a "sharing flag" indicating that the associated medical data set is to be shared. If this is the case, the method (the system/the connectivity node 30) proceeds with uploading the medical data sets to cloud platform 40. The sharing flag may, for instance, be set during administrative workflows and may be encoded in the associated information.

In Step S140, the medical data set and/or the associated information are further processed to identify from the medical data set and/or the associated information one or more users 50 entitled to access the respective medical data set. Primarily, it is usually the patient who is entitled to access his own medical data sets. Information concerning the patient may likely be contained in the header of the medical data sets - alongside other metadata concerning the data source (what system the image data came from), anatomical type that the image data corresponds to (e.g., chest, abdomen, head), anatomical view of the image data (e.g., posterior, anterior, lateral). Accordingly, connectivity node 30 may be configured to look at the header of the medical data sets to identify entitled users 50. As indications pertaining to entitled users 50 may also be hidden in the body of the medical data set connectivity node 30 may further be configured to search the entire medical data sets, for instance, by relying on semantic search tools or on optical character recognition (OCR). While it can be assumed that the patient (as entitled user 50) is derivable from the medical image data, it is considerably less likely that all of the other entitled users are likewise recorded in the medical data sets. This is because other entitled users 50 such as other specialist physicians usually only come into play after the medical data sets have been recorded, or are systematically not recorded in the medical data at all (as is often the case for referring physicians). Typically, this information is recorded in electronic patient records as one example of associated information and not in the medical data sets. Accordingly, connectivity node 30 may further be configured to search the associated information for (additional) entitled users 50. As in the case of processing the medical data sets, the system may be configured to look in the header (if any) as well as in the body of the associated information.

While it may be beneficial to process both, the medical data sets as well as the associated information, in order to ensure that all entitled users 50 are correctly identified, this is not mandatory, however. It should be noted that the method can also be put into practice by only reading (processing) either the medical data sets or the additional information for identifying the entitled users 50. Whether or not this is feasible depends on the specific requirements and circumstances in the local environment 2. If, for instance, a hospital keeps a complete record of all entitled users 50 in the associated information, it is enough to only evaluate the associated information. Moreover, if it is only required to share the medical data sets with the patients (and provided that this information is systematically recorded in the medical data sets), it might also be sufficient to only process the medical data sets for identifying the entitled users 50 (without taking the associated information into account at all) .

Once the entitled users 50 have been identified, the system is configured to retrieves additional user information of the entitled users 50 in step S150. This additional user information is subsequently used to grant the entitled users 50 access to "their" medical data sets in the cloud platform 40 (step S160) and eventually complete the process of sharing the medical data sets. The additional user information is required since it is generally not possible to forward the medical data sets based on the mere indication of the entitled user 50. Rather, the system requires additional user information to do so. The additional user information may comprise, an email or postal address of the entitled user 50, whether or not the entitled user 50 has an account for cloud platform 40 and, if so, what the account details are, the entitled user's telephone number, preferred ways of notification, etc. Such additional user information may be recorded in the medical data sets. However, this is unlikely. A better source of information in this regard is the associated information where data pertaining to the administration of the hospital is recorded. Accordingly, the system may be configured to retrieve the additional user information by processing (i.e., reading or searching) the associated information. As an alternative or in addition to that, connectivity node 30 may be configured to retrieve the additional user information from one or more separate user data repositories 60 such as electronic address books. To this end, connectivity node 30 may be configured to query the user data repositories 60 based on the name or any other suitable identifier for the entitled user 50.

In step S160, the additional user data is used to grant the entitled users 50 access to the respective medical data sets in cloud platform 40. This may involve transmitting to the cloud platform 40 an information pertaining to the entitled users 50 for the respective medical data set. This may involve determining whether or not the entitled users 50 have an account with the cloud platform 40. If this is the case, the respective medical data sets are linked to the corresponding user account of the entitled user 50 and are made accessible to the entitled user 50 via the user account. In addition, it may be communicated to the entitled user 50 that new medical data sets have been uploaded to his account, e.g., via email or SMS.

If the entitled user 50 does not have a user account in cloud platform 40, temporary access to cloud platform 40 may be generated for the entitled user 50 via a user portal to the cloud platform 40. To this end, the entitled user 50 may be provided with an URL for accessing the medical data via the user portal. The URL may be sent to the entitled user 50 via email, for instance.

In addition to that, step S160 of granting access may comprise generating a passcode for accessing the respective medical data sets and forwarding the passcode to the entitled users 50. If the entitled users 50 want to access the medical image data, they may subsequently be requested to insert the corresponding passcodes. Upon receipt of the passcodes, the system may be configured to check if the code entered by the entitled user 50 matches the generated passcode. If this is the case, the entitled user 50 is granted access to the medical data sets. Preferably, passcodes are forwarded to the entitled users 50 using different communication channels than for notifying the entitled users 50 that new content has been assigned to the corresponding cloud storage account and/or for forwarding the URL for temporary access.

As indicated in connection with Figures 1 and 2, the steps of processing S140 and the step of retrieving the additional user information S150 are preferably performed locally at connectivity node 30. Step S160 of granting access may likewise be performed predominately at connectivity node 30. Actions that are naturally performed at cloud platform 40, such as the receipt and cross-checking of the passcodes, the action of linking the medical image data to the user account and so forth may be initiated and controlled by connectivity node 30. This may involve forwarding to cloud platform 40 all the relevant information and corresponding instructions necessary for these actions by connectivity node 30. This involves information about the entitled users 50, information about user accounts, information about the preferred ways of access and the like. In other words, the retrieved additional user information may be forwarded to cloud platform 40. As an alternative, only an indication about the entitled users 50 may be forwarded to cloud platform 40, and cloud platform 40 may be adapted to retrieve the additional user information on its own motion, for instance, by accessing a separate user data repository linked to cloud platform 40. Under such circumstances, steps S150 and S160 would be performed predominately at cloud platform 40. As yet a further, intermediate alternative, only step S160 may be predominately performed at cloud platform 40 on the basis of the outcome of steps S140 and S150 as forwarded to cloud platform 40 by connectivity node 30.

In the following, two exemplary workflows for sharing medical data sets according to embodiments are described. The following is to be construed by ways of example and not as limiting the disclosure. For the sake of easy reference, several (optional) steps as described above have been left out (but may likewise be integrated into the examples). Further, individual steps of the following examples may also be combined with one another.

The first exemplary workflow is directed to sharing a medical data set with an entitled user 50 having an account in cloud platform 40, which is typically the case for referring physicians, for instance. Moreover, it is assumed that the medical data set is formatted in the DICOM-format and that the associated information is retrieved from a HIS/RIS system over a network communicating in the HL7 format.

Under these circumstances, sharing the medical data set uploaded into cloud platform 40 with a referring physician (as the entitled user 50) in short may be done by automatically mapping the referring physician mentioned in the medical data set to his user account with cloud platform 40. In this regard, the physician's email address may be used to link the referring physician's identity within the hospital to his user account in cloud platform 40. More specifically, this may translate into the following steps:
a) A radiologist or technician sends a medical data set (in the form of DICOM data) to a locally installed connectivity node 30 where it is received (according to step S110 as defined above). For that purpose, connectivity node 30 provides a DICOM Application Entity Title, which can be configured as send destination in every PACS, scanner or DICOM node. Relying on a dedicated DICOM Application Entity Title enables the method to automatically receive the medical data sets.
b) The medical data sets are uploaded to the cloud platform 40 (according to step S120 as defined above).
c) In parallel, connectivity node 30 listens to the HL7 and/or FHIR data stream in the hospital from HIS/RIS thereby receiving associated information corresponding to the medical data sets (according to step S130 as defined above).
d) The referring physician is read from the DICOM header of the uploaded medical data set and/or from the associated HL7 message which has the same accession number as the DICOM medical data set (according to step S140 as defined above).
e) The referring physician's email address and by the additional user information is read from the associated HL7 ADT message or, if not available, from user data repository 60 (according to step S150 as defined above).
f) The email address of the referring physician is mapped to the referring physician's account in cloud platform 40 and the medical data sets are shared with the account of the referring physician in cloud platform 40 (according to step S160 as defined above).

For the second example, it is assumed that the entitled user 50 is a patient, who does not have a user account in cloud platform 40. While points a)-c) correspond to the first example, points d)-f) are slightly adapted as follows.
d) Identifying attributes of the patient such as a patient name, patient ID, birthdate, sex or the like are read from DICOM header of the received medical data set and/or from the associated information in the form of the HL7 and/or FHIR data stream in the hospital from HIS/RIS (according to step S140 as defined above).
e) The patient's email address is read from the associated HL7 ADT message or, if not available, from the cloud address book (according to step S150 as defined above).
f) A unique passcode for the sharing activity is created and forwarded to the patient. Further, a link (such as a URL) to the medical data set is generated and, likewise, forwarded to the patient. This is done via email. The unique passcode is provided via another communication channel than email to the patient, e.g., via SMS to the phone number of the patient or a printout given to the patient at the hospital. Patients can then get access to their medical data sets by using a patient portal to cloud platform 40. The patient portal can be accessed only with the specific link (URL) and by the unique passcode (according to step S160 as defined above).

The method steps S110 to S160 together with their optional or alternative extensions as described above may be performed when a corresponding computer program product is loaded into the memory of the computing unit (processor 31). The computing may be local in the sense that the program runs on a dedicated local processor such as in the connectivity node 30 optimized for the particular purpose. Alternatively, the program may run on premises on a local server or cluster of sub-units within the local environment 2. Finally, at least parts of the program may also be executed in the cloud platform 40, in particular, if they pertain to the details of granting access to the medical data sets uploaded to the cloud. In this regard, processors or computing sub-units inside the local environment may prompt external components to execute the respective method steps.

Wherever meaningful, individual embodiments or their individual aspects and features can be combined or exchanged with one another without limiting or widening the scope of the present invention. Advantages which are described with respect to one embodiment of the present invention are, wherever applicable, also advantageous to other embodiments.

The following points are also part of the disclosure:
1. Computer-implemented method for sharing medical data sets,
   the method comprising the steps of:
   - receiving medical data sets from a medical system;
   - receiving associated information from a database separated from the medical system, wherein the associated information provides information associated to the medical data sets;
   - uploading the medical data sets to a cloud platform;
   - identifying one or more entitled users by processing the medical data sets and the associated information, the entitled users being entitled to access a respective medical data set;
   - retrieving additional user information of the entitled users;
   - granting the entitled users access to the respective medical data set in the cloud platform on the basis of the additional user information.
2. Method according to 1, further comprising the step of
   determining for the received medical data sets whether or not they shall be shared with all or part of the entitled users on the basis of the medical image data, the associated information and/or the additional user information; and
   wherein in the step of uploading, an uploading of the medical data sets is only performed if it has been determined that they shall be shared with the entitled users.
3. Method according to any of the preceding points, wherein in the step of uploading, medical data sets are only uploaded if at least one entitled user has been identified for the respective medical data set.
4. Method according to any of the preceding points, wherein the step of processing comprises
   accessing and reading the associated information to identify one or more entitled users from the associated information; and
   accessing and reading the medical data sets to identify one or more entitled users from the medical data sets.
5. Method according to any of the preceding points, wherein the step of processing comprises
   reading metadata contained in the medical data sets and/or the associated information.
6. Method according to any of the preceding points, further comprising the step of
   querying the database for the associated information after receipt of the medical data sets.
7. Method according to any of the preceding points, further comprising the step of
   associating to the medical data sets the corresponding associated information.
8. Method according to any of the preceding points, further comprising the steps of
   respectively extracting, from the medical data sets, unique identifiers; and
   querying the database for the associated information on the basis of the unique identifiers.
9. Method according to any of the preceding points, wherein the associated information is formatted according to the HL7 and/or FHIR standard.
10. Method according to any of the preceding points, wherein the step of receiving medical data sets comprises receiving the medical data set via an interface, wherein the interface is assigned an identifier, in particular, a DICOM Application Entity Title.
11. Method according to any of the preceding points, wherein
   the entitled users comprise patients, referring physicians and/or referring hospitals or practices.

## Claims

1. Computer-implemented method for sharing medical data sets,
the method comprising the steps of:
- receiving (S110) medical data sets from a medical system (10) ;
- receiving (S130) associated information from a database (20) separated from the medical system (10), wherein the associated information provides information associated to the medical data sets;
- uploading (S120) the medical data sets to a cloud platform (40);
- processing (S140) the associated information and the medical data sets to identify one or more entitled users (50), the entitled users (50) being entitled to access a respective medical data set;
- retrieving (S150) additional user information of the entitled users (50);
- granting (S160) the one or more entitled users (50) access to the respective medical data set in the cloud platform (40) on the basis of the additional user information.

2. Method according to claim 1, wherein
- the medical data sets are formatted according to a first communication standard, and
- the associated information is formatted according to a second communication standard,
- wherein the first communication standard is different from the second communication standard.

3. Method according to any of the preceding claims, wherein, in the step of retrieving (S150):
- the additional user information is extracted from the associated information, and/or
- the additional user information is retrieved from the database (20), and/or
- the additional user information is retrieved from a separate user data repository (60).

4. Method according to any of the preceding claims, wherein the additional user information comprises:
- an email address of the one or more entitled users (50),
- a phone number of the one or more entitled users (50),
- an account information of the one or more entitled users (50) in the cloud platform (40),
- an address of the one or more entitled users (50),
- and/or any combination thereof.

5. Method according to any of the preceding claims, wherein the step of granting access (S160) comprises:
- mapping the one or more entitled users (50) to respective accounts of the one or more entitled users (50) in the cloud platform (40) on the basis of the additional user information; and
- making the respective medical data set accessible to the one or more entitled users (50) via the respective accounts.

6. Method according to any of the preceding claims, wherein the step of granting access (S160) comprises:
- establishing whether or not the one or more entitled users (50) have a respective account in the cloud platform (40) on the basis of the additional user information.

7. Method according to any of the preceding claims, wherein the step of granting access (S160) comprises:
- respectively generating one or more URLs for the one or more entitled users (50) with which the one or more entitled users (50) can access the respective medical data set in the cloud platform (40); and
- respectively forwarding the one or more URLs to the one or more entitled users (50) on the basis of the additional user information.

8. Method according to any of the preceding claims, wherein the step of granting access (S160) comprises:
- respectively creating unique passcodes for the one or more entitled users (50),
- forwarding the unique passcodes to the one or more entitled users (50) on the basis of the additional user information; and
- granting access to the respective medical data set in the cloud platform (40) upon receipt of the unique passcodes from the one or more entitled users (50).

9. Method according to any of the preceding claims, wherein the medical data sets are formatted according to the DICOM standard, and/or wherein the associated information is formatted according to the HL7 and/or FHIR standard.

10. Method according to any of the preceding claims, further comprising the steps of
- extracting, from the medical data sets, a unique identifier; and
- associating, to the medical data sets, the corresponding associated information on the basis of the unique identifier.

11. System for sharing medical data sets comprising
- an interface unit (31) configured to communicate with
• a medical system (10);
• a database (20) separate from the medical system (10); and
• a cloud platform (40);
wherein the system further comprises
- a computing unit (32) configured to
• receive medical data sets from the medical system (10) via the interface unit (31);
• receive associated information from the database (20) via the interface unit (31), wherein the associated information provides information associated to the medical data sets;
• process the medical data sets and the associated information in order to identify one or more entitled users (50), the one or more entitled users (50) being entitled to access a respective medical data set;
• retrieve additional user information of the one or more entitled users (50);
• upload the medical data sets to the cloud platform (40) via the interface unit (31); and
• grant the entitled users (50) access to the respective medical data set in the cloud platform (40) on the basis of the additional user information.

12. System according to claim 11, wherein the system comprises a DICOM Application Entity Title.

13. Environment for sharing medical data sets comprising:
- the system according to any of claims 11 or 12, and
- the medical system (10), the medical system (10) being configured to acquire and/or store and/or transmit the medical data sets.

14. Computer program product comprising program elements which induce a computing unit (32) of a system for sharing medical data sets to perform the steps according to the method of any of claims 1 to 10, when the program elements are loaded into a memory of the computing unit (32) .

15. Computer-readable medium on which program elements are stored that are readable and executable by a computing unit (32) of a system for sharing medical data sets, in order to perform steps of the method according to any of claims 1 to 10, when the program elements are executed by the computing unit (32).
